# EUROPEAN PATENT APPLICATION

(11) **EP 4 534 052 A1**
(43) Date of publication of application: **09.04.2025**
(21) Application number: 23811224.7
(22) Date of filing: 11.05.2023
(51) Int. Cl.: A61F 6/04

(54) **COUPLING FOR RETAINING AND SECURING MALE OR FEMALE CONDOMS THAT IS EFFECTIVE AGAINST SEXUALLY TRANSMITTED INFECTIONS**

(30) Priority: 27.05.2022 ES 202230890 U; 21.02.2023 ES 202330276 U
(71) Applicant: Mym Global Innovations, S.l., 31892 Zuasti (ES)
(72) Inventor: BORDÉS MAYO, Manuel, 31892 Zuasti (ES)
(86) International application number: PCT/ES2023/070304
(87) International publication number: WO 2023/227810

(57) **Abstract**

The present invention comprises a body (1-101) for adapting to the male or female anatomy, said body extending the genital contact barrier with respect to the male or female condom (102), such that a neck (2) or flexible annular sector (103) on which the male or female condom (102) can be coupled is established on said body (1-101), the neck (2) or flexible annular sector (103) externally having an outer perimeter sealing lip (3) or flexible annular appendage (104) on which the end ring or final portion of the body of the male or female condom (102) is tightly coupled, the neck (2) having an inner perimeter sealing lip (5), being complemented with an elastic ring (105) that overlaps the assembly formed by the flexible annular sector (103) or neck (2) and the condom.

## Description

### OBJECT OF THE INVENTION

The present invention relates to a coupling device for retaining and securing male and female condoms, which has a flexible annular appendage or perimeter sealing lip especially designed to attach to the condom, to secure or grip its end rim and form an airtight and safe assembly with said condoms, by which two objectives are obtained: securing and retaining the condoms in their correct position during sexual intercourse, avoiding their involuntary or accidental removal in addition to avoiding the transmission of sexually transmitted diseases or infections, as this coupling device prolongs the protection of these condom sheaths by means of a segment that adapts to the male or female anatomy, protecting and insulating the area of genital skin susceptible to infecting or being infected by a Sexually Transmitted Infection (STI) or Sexually Transmitted Disease (STD), an area of genital skin that the current range of condoms does not protect in any way. The coupling device also allows the user to attach the condom simply, practically and safely without damaging or jeopardising the condom.

The object of the invention is, therefore, to provide and promote new products that reinforce, with total guarantee, safety in sexual activities in order to:
1.- Help people affected by any type of sexual condition, infection or disease to continue to have a 'dignified and safe' sexual life while being treated by different specialists and at the same time avoid spreading to or infecting third persons.
2.- Help in the fight against and eradication of STIs/STDs (a worldwide public health problem) by incorporating these means into the market together with all the information on the reality of the dangers, risky sexual practices and the correct way to avoid them.

This is because the present invention eliminates the risks of transmission of sexual infections that occur through direct skin-to-skin contact of genital areas susceptible to contracting or spreading an STI/STD, whether or not there is penetration, and which are not covered by the current range of condoms presently on the market, as such skin-to-skin genital contact has been shown to be contagious and transmitter of STI/STDs, as affirmed by the World Health Organization (WHO), as well as the health risks that could result from unintentional or accidental removal of the condom during sexual intercourse.

The device of the invention is therefore intended to prevent the transmission of infections and/or sexual diseases of all kinds that occur through 'penetration', with special emphasis on those that are transmitted by direct skin-to-skin genital contact regardless of the existence or not of skin lesions.

### BACKGROUND OF THE INVENTION

According to data published on 6 June 2019 by the World Health Organization (WHO), more than 1 million people aged 15 to 49 are infected every day with a sexually transmitted infection (STI) that can be cured. In total, more than 376 million new cases are reported every year.

Dr Peter Salama, Executive Director of WHO's Division for Universal Health Coverage - Life Course stated: "We are witnessing a worrying lack of progress in the fight to stop the spread of sexually transmitted diseases around the world".

To better understand this global public health issue and the solution provided by this new invention, we will focus on explaining just one aspect. HPV or the Human Papillomavirus.

According to the World Health Organisation (WHO), HPV or human papillomavirus is the most common cause of viral infection of the reproductive apparatus. Most sexually active women and men will become infected at some point in their lives, and some people will develop recurrent and persistent infections that lead to cancer. Certain types of HPV infection also cause some cancers of the anus, vulva, vagina, penis and oropharynx. The WHO recognises that cervical cancer is a public health problem.

The WHO asserts that most men and women become infected soon after becoming sexually active and that HPVs are transmitted through sexual contact, even though penetrative sex is not necessary for transmission to occur. "Direct skin-to-skin contact in the genital area is a recognised mode of transmission of the disease".

All women are at risk of HPV infections becoming chronic and precancerous lesions progressing to invasive cervical cancer (ICC), indeed, worldwide, cervical cancer (CC) is the fourth most common cancer among women. It is estimated that in 2018 there were 570,000 new cases, accounting for 7.5% of female cancer mortality.

There are more than 100 types of HPV of which 40 were detected in the genital apparatus. The following 15 types of HPV were classified as high-risk: HPV16, HPV18, HPV31, HPV33, HPV35, HPV39, HPV45, HPV51, HPV52, HPV56, HPV58, HPV59, HPV68, HPV73 and HPV82. Types HPV26, HPV53 and HPV66 were classified as possible high risk, and 12 types were classified as low risk: HPV6, HPV11, HPV40, HPV42, HPV43, HPV44, HPV54, HPV61, HPV70, HPV72, HPV81 and CP6108.

There are currently three vaccines that target the following genotypes:
- Cervarix, GlaxoSmithKline contains VLPs of the high-risk genotypes HPV16 and HPV18.
- Gardasil, MSD contains VLPs of the high-risk genotypes HPV16 and HPV18 and of the low-risk genotypes HPV6 and HPV11.
- Gardasil 9, MSD contains VLPs of the high-risk genotypes HPV16, HPV18, HPV31, HPV45, HPV52, HPV58 and of the low-risk genotypes HPV6 and HPV11.

The remaining genotypes do not have a vaccine. It is important to note that the vaccine does not serve as a treatment for serotype infections already contracted prior to administering the vaccine. Vaccines should be administered at an early age (recommended age: 12 years), before having sexual relations. In addition, the vaccine currently has a political/economic/social connotation that makes it inaccessible to many people in the world, so prevention remains the most effective tool.

In society today, there is an increasing demand for information about STIs/STDs. "In Spain, since 2000, an increase in the number of STIs/STDs under epidemiological surveillance has been observed". Professionals should incorporate information on safe sex both in consultations and in group health education activities, with the aim of promoting sexual and reproductive health measures in the population.

Healthy practices to avoid STIs/STDs are based on safer sex behaviour, including the use of condoms. Condoms, even when used correctly, are not effective in preventing STIs/STDs because they are not designed to cover the area of genital skin susceptible to contracting or transmitting STIs/STDs. Contact with this area of skin is in itself a source of transmission, as recognised by the WHO, whether or not there are lesions, as in the case of human papillomavirus (HPV).

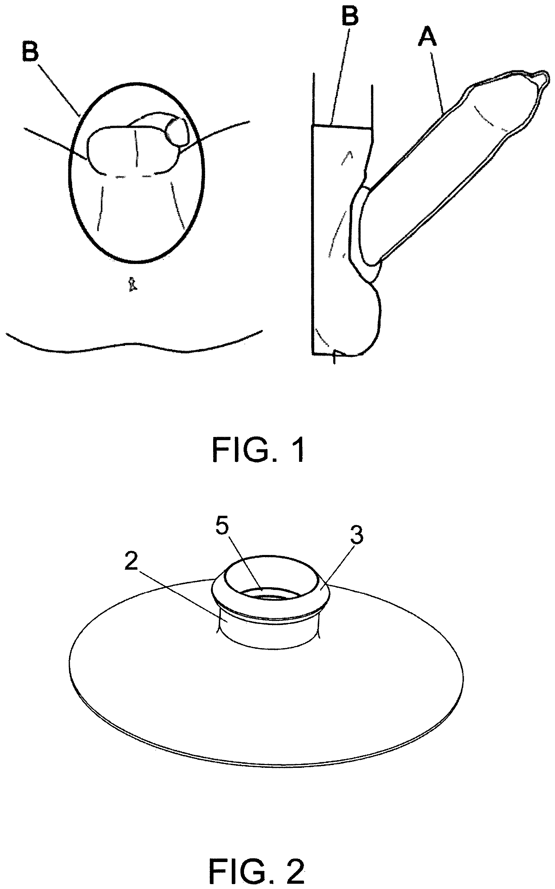

As can be observed in Figure 1, the area (A) of skin covered by conventional condoms leaves an area (B) exposed to Sexually Transmitted Infections, and as the female genitalia are much larger than the male genitalia so also is the area of genital skin where both meet during sexual intercourse.

It is important to note that if you contract an STI/STD, you are more likely to contract HIV than someone who does not have an STI. This is because the same behaviours and circumstances that can put you at risk of contracting an STI/STD can also put you at greater risk of contracting HIV. In addition, if you have a sore or wound on your skin caused by an STI/STD, you are at risk of becoming infected with HIV.

Having outlined and explained the shortcomings of preventing STI/STD that exist with the current range of condoms that we effectively resolve with this innovation, it must be mentioned that there exist male erotic garments intended to be used in combination with condoms about which it is worth noting:
- These are erotic garments, not airtight extensions developed to be attached to and extend the protection of the conventional male condom sheath safely during sexual intercourse.
- They have not been created, developed or designed with a specific and/or concrete use, aimed exclusively at preventing Sexually Transmitted Infections during sexual intercourse.
- Consequently, they are not medical-health products regulated by the HEALTH department nor by the strict legal regulations that authorise them, namely the European Community Directive 93/42/CE and the European Regulation (EU) 2017/745, so they cannot be found in the PHARMACEUTICAL sector or at condom points of sale.
- One of the purposes of the genital contact barrier object of this invention is to guarantee TEAR-RESISTANT safety (93/42/CE) and, therefore, the non-transmissibility of STI/STD whereas these garments that exist in the market, whose application is intended as underwear or erotic garments, are easy to damage, passing all the responsibility to the final consumer.
- The extensions that can be obtained from the present invention must guarantee the HYGIENIC/SANITARY criteria (93/42CE), while these erotic products remain outside all sanitary regulations passing all the responsibility to the end user.
- In these garments, as an element that is combined with a condom, the area of confluence between these elements in no way determines an airtight, optimal and safe barrier that avoids the risks of contracting an STI/STD. The present invention develops the airtight and safe coupling of the condom to these extensions or vice versa, as indicated in the 'EXPLANATION OF THE INVENTION' section of this document.
- Furthermore, the condom in these types of garments could accidentally or unintentionally become loose or even come off the penis during sexual intercourse, with the consequent risks that this entails, a problem which is also developed, solved and explained in the section 'EXPLANATION OF THE INVENTION'.
- As garments, whether for underwear or as erotic garments, they are not required to display/provide/include an information sheet together with the product for the final consumer indicating how to use it correctly and safely during sexual intercourse, whereas medical/health products, as the present invention intends to be, are obliged to provide such information together with the product.

Finally, it should be stressed that the above mentioned issues are equally applicable to both male and female condoms.

### DESCRIPTION OF THE INVENTION

The proposed invention satisfactorily solves the above-mentioned problem by using a simple but effective solution.

More specifically, the device of the invention consists of a coupling means equipped with a flexible annular appendage or perimeter sealing lip that allows the attachment and airtight fastening to the conventional male or female condom, which prolongs the protection of the condom sheath and therefore of the sexual barrier whose main purpose is to cover and prevent contact of the genital skin exposed to an STI/DSTD, in addition to keeping the condom in place during sexual intercourse, preventing its accidental or involuntary removal.

The coupling variant intended for male condoms consists of an elastic, pliable, open neck, dimensionally shaped and adapted to the base of the penis, with the special feature that the neck has a flexible annular appendage or perimeter sealing lip on both the outside and the inside which is angled to create airtightness and hermeticity by means of pressure, developed so that the user can put on the condom of his choice, whether internally or externally, in a way that is simple, practical and in total safety, in other words, the neck can be fitted over the penis already covered with the conventional condom or the condom can be placed over the neck and penis.

In both cases of the male version, (whether positioned internally or externally) the sealing lip will exert pressure, airtightness and hermeticity that prevents the involuntary or accidental removal of the condom, thus offering impermeability and an assemblage (coupling and condom) suitable and safe for sexual intercourse since, in addition to preventing accidental or unintentional removal of the condom, this assemblage extends the protection of the condom sheath to the area of genital skin that is susceptible to infecting or being infected by a Sexually Transmitted Infection (STI) or Sexually Transmitted Disease (STD). Depending on the model used, for additional reinforcement and safety, the surfaces may have non-slip areas by way of a rough relief.

In this way, in the male variant, the device of the invention together with the conventional male condom will form an airtight and optimal combination for sexual intercourse, a device that will be attached to the user's body by means of adhesion, suction, straps, tapes, elastic elements, etc. or by attaching to or incorporating into fabric, textile, erotic or sexual garments by means of Velcro or other conventional systems suitable for this purpose.

In turn, in the variant of the device intended to be applied to female condoms, the coupling of the invention is made up of a main body for adaptation to the female anatomy which provides an extension of the barrier of genital contact with respect to the condom, having a flexible annular sector or neck which has a flexible annular appendix or outer perimeter sealing lip which is angled towards the wall of the neck, facing its base, which defines a degree of adjustment, impermeability and pressure given the increased diameter, a flexible annular sector on which the female condom is intended to be attached, annular element comprising an external perimeter flange of a larger dimension than that of the condom rim, which makes it necessary to stretch the rim of the condom to fit onto said flange and so retain it in place given the increased diameter to which it must be adjusted with the consequent increase in pressure.

The flange may have different configurations to ensure effective retention of the rim of the condom, defining an external upper perimeter flange that may be perpendicular to the axial axis of this annular element, or even adopt a certain angle of inclination, both obtuse and acute.

This geometry prevents the formation of labyrinths, which facilitates cleaning.

The annular sector thus described has a curvature that is similar to the female anatomy, which, together with the elasticity of the material allows for a good and comfortable fit.

At the same time, the edges which are described in the area where the condom and the penis pass through have been designed to be markedly rounded.

To further increase the degree of safety of the device, provision has been made so that the main body may optionally be fitted with an elastic ring which overlaps the assemblage formed by the annular sector and the condom, so it is necessary to stretch this ring to fit into place. This ring has an inner ribbing designed to fit on the perimeter flange of the annular sector of the main body, preventing it from moving and thus locking the rim of the condom. This ring can also be applied to the male version.

This ring will preferably be attached to the body to facilitate its positioning and prevent it from getting lost.

As mentioned above, the main body includes a segment that increases the area of skin covered, improving the safety, comfort and aesthetics of the device.

The device can be used independently, attached, incorporated or tailored to a garment so that it is integrated as part of the garment or included in other types of sexual paraphernalia, toys and sexual stimulators, regardless of the means of attachment or fastening to the female body.

In order to make this invention attractive also to the female sector and to extend its use more effectively in our attempts to assist in the campaign to eradicate STDs, this invention is capable of incorporating female stimulation elements onto both the neck and its base or prolongation.

In this way, the device of the invention in any of its versions, together with the condom and the garment or sexual barrier, will form an airtight and optimal assemblage for sexual practice, a device that will be attached to the user's body by itself, by means of adhesion, suction, straps, tapes, elastic elements, etc. or attached, incorporated or form an integral part of fabrics, erotic or sexual garments, including toys or sexual stimulators, by means of Velcro or other conventional systems suitable for this purpose Irrespective of the size, dimensions or shapes of the extension to the sexual barrier, whether for the male or female version, it may have an opening in the anal area or be left uncovered to allow anal penetration.

The device thus described may be made of latex, plastic products, waterproof products or any other suitable material, in particular those required by health regulations, which reliably ensure the non-transmission of STIs/STDs in the genital areas.

In both versions described, the device can be complemented with lubricants, aromas or flavours, and are available in various colours, etc.

In this way, penetration can be performed with total guarantee, without risk of contracting or transmitting sexually transmitted diseases, which makes this innovation a unique and effective tool to solve part of the current public health problem derived from STIs/STDs.

With this configuration, a genital contact barrier is obtained that will prevent direct skin-to-skin contact in the genital areas exposed to STIs/STDs during sexual intercourse. Moreover, this new range of safe sexual barriers will not only help to prevent the risk of contagion or transmission of sexual diseases, but will also help people already infected to have a dignified sexual life while being treated by different specialists without the risk of infecting third persons and without having to abstain from sexual relations with the associated psycho-emotional problems such as: Stress, problems and/or mistrust in the couple, depression, rejection of sex, lack of self-esteem, insecurity, fear of possible consequences such as the development of cancer, new lesions and even the rupture of the relationship that unites the couple/marriage, etc. that damage the practice and benefits of sex.

This new generation of sexual barriers also aims to address the global public health problem by eliminating condom deficiencies and thus the cause of transmission. The implementation of these barriers will help awareness-raising, education, prevention and eradication of STIs/STDs, since one of the most important problems is the lack of knowledge among the population of the existence of these areas of risk of transmission of sexual infections and diseases that are not covered by condoms, more specifically, the direct contact of genital skin susceptible to transmitting and/or being infected by an STI/STD from the infected person who, in the vast majority of cases, does not show any symptoms.

### BRIEF DESCRIPTION OF THE DRAWINGS

To complement the description given below and in order to help towards a better understanding of the characteristics of the invention, in line with a preferential example of a practical embodiment thereof, a set of drawings is attached as an integral part of said description, for illustrative and non-limiting purposes:
Figure 1.- Shows two views of the male area exposed to STIs/STDs obtained with a conventional male condom, taking into account that the female sexual organ, not represented in the drawing, is much larger than the male and so also the area of contact during sexual intercourse.
Figure 2.- Shows an elevated perspective of the dual-use coupling device for male condoms, made in accordance with the object of the present invention.
Figure 3.- Shows an overhead view of the device in figure 2.
Figure 4.- Shows a diametrical section view of the device in figures 2 and 3, together with a close-up showing the internal and external means of securing the condom.
Figure 5.- Shows a view similar to that of figure 3, but in which the barrier is applied to a 'harness' type garment.
Figure 6- Shows an example of a practical embodiment of the male version, in which the genital contact barrier is applied to a textile, erotic or sexual garment.
Figure 7.- Shows a perspective view of a coupling device for retaining and securing female condoms, effective against sexually transmitted infections, made in accordance with the object of the present invention, in a variant in which the device is incorporated on a female garment.
Figure 8.- Shows a front elevation view of the assemblage of figure 7, with the ring duly attached to the annular sector.
Figure 9.- Shows a low-angle view of the assemblage of figures 7 and 8.
Figure 10.- Shows a rear elevation view of the assemblage of figures 7, 8 and 9.
Figure 11.- Shows a profile view of the assemblage of figures 7, 8, 9 and 10 with the ring detached from the annular sector.
Figure 12.- Shows a view similar to that of figure 11, but in which the ring is attached to the annular sector.
Figure 13.- Shows a low-angle view of the device of the invention, in a female version in which the device is not attached to a garment, so it is attached directly to the user's skin by its own adhesive nature.
Figure 14.- Shows a sectional view of the assemblage of figure 13, in accordance with the cutting plane D-D shown in said drawing.
Figure 15.- Shows a view similar to that of figure 14, but in which the different operational positions of the female condom are illustrated, from its initial circumference (I), its maximum stretch circumference (M) and its in-place circumference (PC).
Figure 16.- Shows a view similar to that of figures 14 and 15, but in which the female condom is shown in position of use.
Figures 17 and 18.- Show views similar to that of figure 14, but corresponding to variants of the annular sector, and more specifically of its perimeter flange, which may adopt different angles and configurations.

### PREFERRED EMBODIMENT OF THE INVENTION

Given the figures shown above, and in particular figures 2 to 6, it can be seen how the device of the invention, in its male version takes the form of a coupling for joining and attaching the condom, which has a body (1) that prolongs the protection of the condom sheath, based on an elastic, pliable and open neck (2), adapted in shape and dimension to the base of the penis, which allows this neck to fit over the penis once it is covered with the conventional condom, in which case the flexible annular appendage or inner sealing lip will exert a degree of pressure and hermeticity between the neck, the condom and the penis, preventing accidental or involuntary removal of the condom, as well as allowing the condom to be placed over the penis and neck where the flexible annular appendage or outer sealing lip lodges between the neck and the condom, exerting greater pressure and hermeticity given the increased diameter which also prevents the involuntary or accidental removal of the condom.

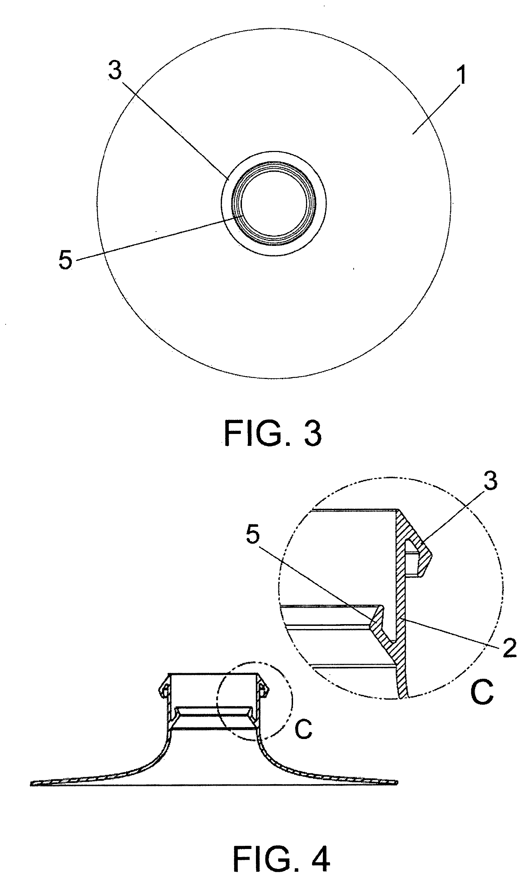

To this end, as can be seen in the close-up of figure 4, externally the neck (2) includes a flexible annular appendage or outer perimeter sealing lip (3) that is angled towards the wall of the neck (2), facing towards its base, which determines a degree of adjustment, pressure and hermeticity between the neck and the condom given the increased diameter, together with the appropriate adaptability of the appendage or lip to both surfaces.

Figure 4 equally shows how the interior part of the neck (2) also includes a flexible annular appendage or inner perimeter sealing lip (5) which is angled towards the wall of the neck (2), this time facing towards its upper aperture, which establishes a degree of greater adjustment, pressure and hermeticity together with the appropriate adaptability of the lip to both surfaces.

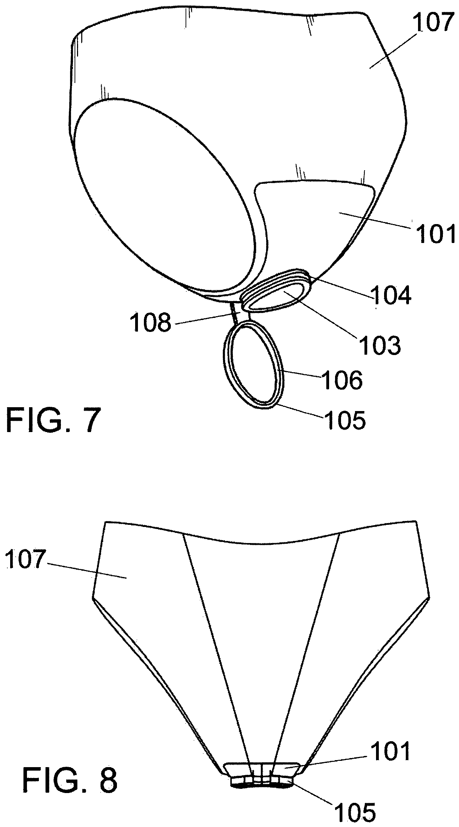

The neck (2) may also have anti-slip areas by way of rough reliefs that help to further increase safety and retention. The neck may be fitted with a ring (105) superimposed on the outside to increase safety, as shown in figure 8 of the female version.

The body (1) is capable of incorporating elements of female sexual stimulation, both at the neck (2) and at its base or prolongation.

The barrier is made of latex, products derived from the plastic sector, impermeable products or any other suitable material, in particular those required at all times in accordance with applicable health regulations and reliably guarantee the non-transmission of STIs/STDs in the genital areas.

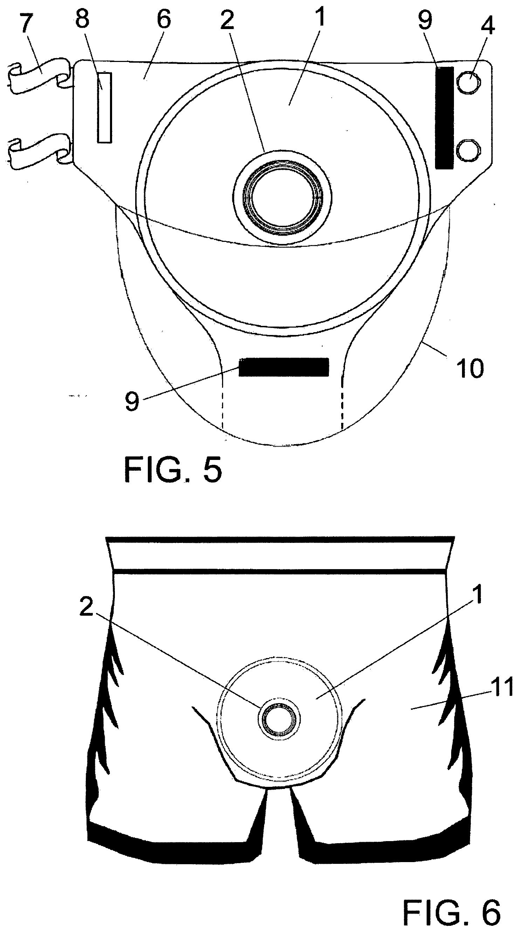

In the example shown in figure 5, a harness (6) is included which may have cut-out openings (8) for passing straps or bands (7), eyelets (4) for passing fastening cords or Velcro (9) for the attachment of other garments or means of fastening.

In this embodiment, the harness (6) includes an area (10) for covering the testicles.

Another of the many possible embodiments of the invention is for the barrier to be directly integrated into a textile, erotic or sexual garment (11), as shown in figure 6, or even in sex toys.

The range of elasticity of the neck (2) will be that which allows appropriate pressure to be applied to the erect penis in order to keep and hold the condom in place with respect to said neck, regardless of whether it is placed internally or externally, without causing discomfort to the user and, in order to adapt to all users safely and comfortably, it may be manufactured in different sizes.

It should be clarified that when we refer to the neck (2) in the male version, we are referring to the flexible/adaptable contour which, in the female version detailed below, mention is made of the term 'flexible annular sector (103)', referring in both cases to the same element but only applied to the human anatomy for which each version is intended.

As shown in Figure 7, the invention is also applicable to female condoms, in which case the device of the invention comprises a lamellar body (101) adaptable to the female anatomy and which constitutes an extension of the genital contact barrier with respect to the female condom (102), a lamellar body (101) in which there is a flexible annular sector (103) on which the female condom (102) is intended to be attached.

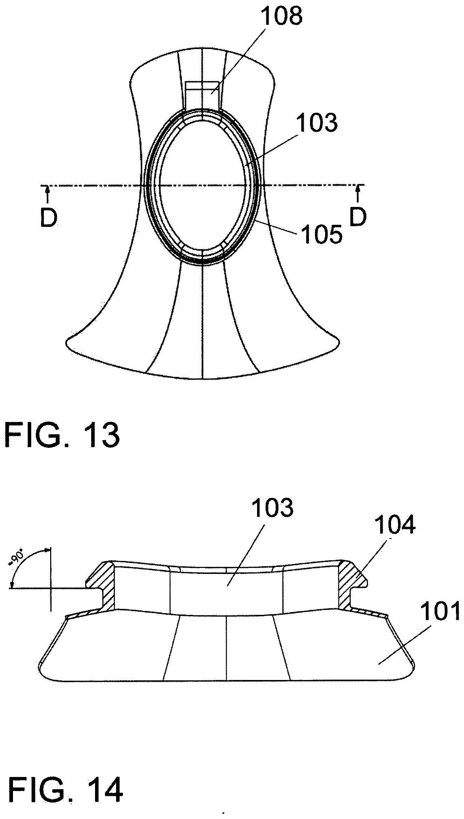

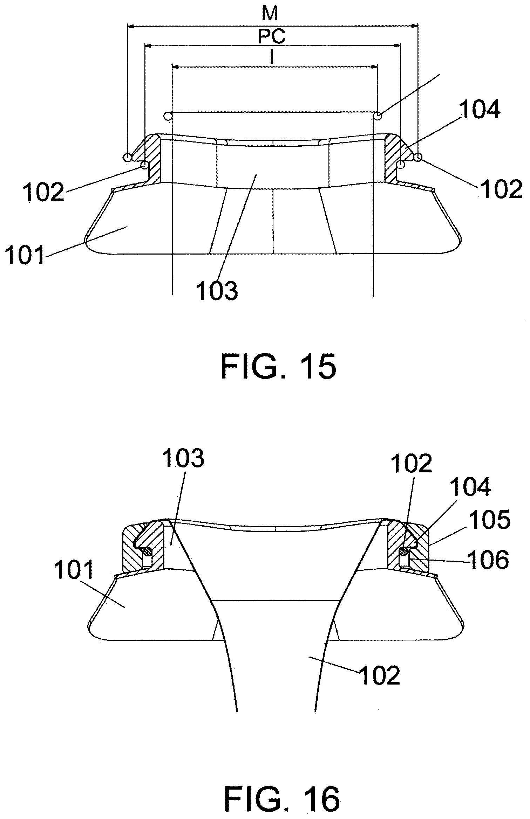

As can be seen in figures 14 to 18, especially in figure 15, the flexible annular sector (103) has an external diameter in consonance with the maximum stretch (M) of the female condom (102), said flexible annular sector (103) having an external perimeter flange or flexible annular appendage (104), with an internal diameter or placement perimeter (PC) greater than the initial perimeter (I) of the free end-rim of said condom.

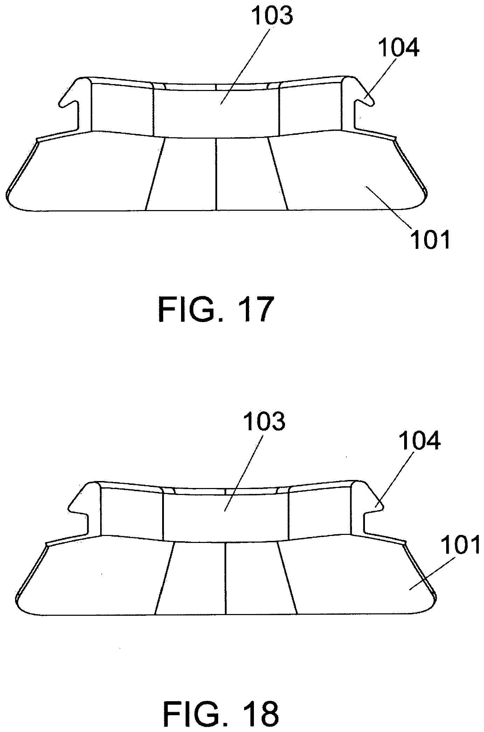

As for the configuration of this outer perimeter flange or flexible annular appendage (104), this may vary according to different design criteria, so it may have an upper rim or flange perpendicular to the axial axis of the annular sector, as shown in figures 14 to 16, or it may have a harpoon-shaped edge as shown in the conformation variants of figures 17 and 18, with its upper edge forming an acute (figure 17) or obtuse (figure 18) angle, with respect to the axial axis of said flexible annular sector (103).

In any case, and as previously mentioned, the edge of the flexible annular sector (103) that comes into contact with the female condom (102) will be distinctly rounded.

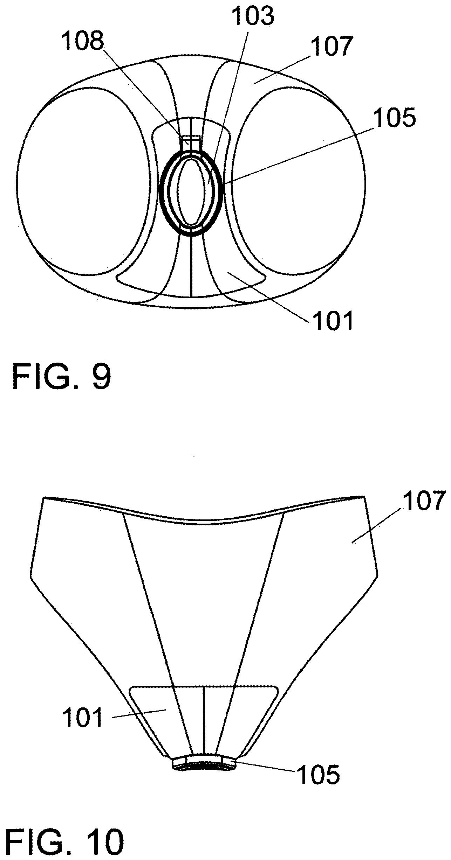

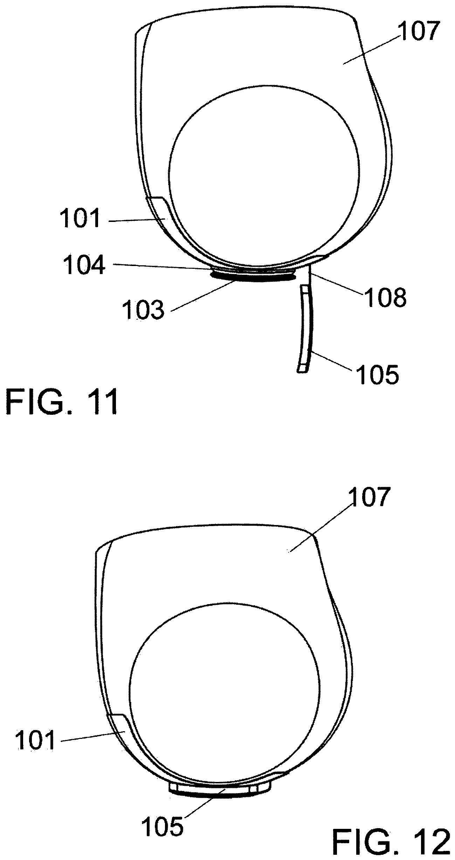

As can be seen from figures 7 to 13, and in particular from figure 16, the device is complemented by a ring (105) of an elastic nature, which is superimposed on the assemblage formed by the flexible annular sector (103) and the female condom (102), so it is necessary to stretch this ring in order to lodge it in position, having an inner rib (106) designed to fit onto the outer perimeter flange or flexible annular appendage (104) as shown in figure 16, preventing the removal of the condom, an element that is equally applicable to the male version, although it is not shown in the drawings.

This ring (105) in the female version is attached to the lamellar body (101) preferably by means of a flexible strip (108) or similar element to facilitate positioning and prevent it from getting lost.

As this device is designed to be integrated into all types of female fabrics, it can even be used as underwear and, to this end, it can be complemented with a cover, not shown in the drawings, for example made of textile material, which adapts to the outer perimeter flange or flexible ring appendage (104), a cover intended to conceal the opening that exposes the female genitalia, a cover that can easily, simply and practically be fitted or removed by the user to position the condom when required or to cover said area.

Finally, the lamellar body (101) can in turn be integrated into other garments (107) in such a way that it is part of the same or is included in other types of garments, fabrics, sexual extensions, toys and stimulators of a sexual nature, independently of the means of fastening or attaching to the female body.

## Claims

1. Coupling for retaining and securing male or female condoms that is effective against sexually transmitted infections, consisting of a body (1-101) adaptable to the male or female anatomy, which provides an extension of the genital contact barrier with respect to the male or female condom (102), a body (1-101) which has a neck (2) or flexible annular sector (103), **characterised in that** said neck (2) or flexible annular sector (103) has a flexible annular appendage (104) or external perimeter sealing lip (3) on which the rubber end-rim of the male or female condom sheath (102) can be fitted, the device being provided with means of attachment to the user's body.

2. Coupling for retaining and securing male or female condoms that is effective against sexually transmitted infections, according to claim 1, **characterised in that** when the coupling is intended for use with male condoms, an annular appendage or inner perimeter sealing lip (5) is additionally incorporated in the neck (2), which is angled towards the wall of the neck (2), oriented towards its aperture, which forms a degree of adjustment, hermeticity and pressure given the reduction of the diameter, providing the body (1) with an extension of the genital contact barrier with respect to the condom.

3. Coupling for retaining and securing male or female condoms that is effective against sexually transmitted infections, according to claim 1, **characterised in that** the coupling is complemented by a ring (105) of an elastic nature, which is superimposed on the assemblage formed by the condom and the flexible annular sector (103) or neck (2).

4. Coupling for retaining and securing male or female condoms that is effective against sexually transmitted infections, pursuant to claims 1 and 3, **characterised in that** the ring (105) in the female version includes means of attachment to the lamellar body (101).

5. Coupling for retaining and securing male or female condoms that is effective against sexually transmitted infections, pursuant to claim 1, **characterised by** having a cover, adapted to the flexible ring appendage (104).

6. Coupling for retaining and securing male or female condoms that is effective against sexually transmitted infections, pursuant to claims 1 and 5, **characterised in that** the cover adapted to the flexible ring appendage (104) is made of textile material.

7. Coupling for retaining and securing male or female condoms that is effective against sexually transmitted infections, pursuant to claim 1, **characterised in that** the body (1) includes anti-slip areas based on relief surfaces.

8. Coupling for retaining and securing male or female condoms that is effective against sexually transmitted infections, pursuant to claim 1, **characterised in that** the means of attachment to the user's body consists of adhesives, suction, straps, tapes, elastic elements or other conventional systems suitable for this purpose.

9. Coupling for retaining and securing male or female condoms that is effective against sexually transmitted infections, pursuant to claim 1, **characterised in that** it is integrated into textile, erotic or sexual garments, including sexual toys or stimulators.

10. Coupling for retaining and securing male or female condoms that is effective against sexually transmitted infections pursuant to claims 1 and 2, **characterised in that** it can incorporate female stimulation elements.

11. Coupling for retaining and securing male or female condoms that is effective against sexually transmitted infections, pursuant to claim 1, **characterised in that** it includes coatings, odorants and/or flavourings.

12. Coupling for retaining and securing male or female condoms that is effective against sexually transmitted infections, pursuant to claims 1 and 9, **characterised in that** the sexual garment in which it is incorporated or forms part, has an aperture which leaves the anus uncovered.
